# EUROPEAN PATENT APPLICATION

(11) **EP 4 711 460 A1**
(43) Date of publication of application: **18.03.2026**
(21) Application number: 24803530.5
(22) Date of filing: 09.05.2024
(51) Int. Cl.: C12N 15/115, A61K 31/7115, A61K 47/54, A61K 47/56, A61K 47/62, A61M 1/36, A61P 3/10, A61P 9/00, A61P 13/12, A61P 21/04, A61P 27/02, A61P 35/00

(54) **DNA APTAMER CAPABLE OF SELECTIVELY BINDING TO SOLUBLE FLT-1 AND CARRIER HAVING SAID DNA APTAMER IMMOBILIZED THERETO**

(30) Priority: 11.05.2023 JP 2023078561
(71) Applicant: TAGCyx Biotechnologies Inc., Tokyo 1530041 (JP); The University of Tokyo, Bunkyo-ku, Tokyo 113-8654 (JP)
(72) Inventor: OWARI, Kensuke, Tokyo 153-0041 (JP); FUTAMI, Kazunobu, Tokyo 153-0041 (JP); IRIYAMA, Takayuki, Tokyo 113-8654 (JP); ARIYOSHI, Yu, Tokyo 113-8654 (JP)
(74) Representative: Schön, Christoph
(86) International application number: PCT/JP2024/017306
(87) International publication number: WO 2024/232421

(57) **Abstract**

The present invention provides a DNA aptamer which is capable of removing soluble FLT-1 (sFLT-1) in the blood more efficiently and specifically, is inexpensive, has uniform quality, and can be used more safely, as a device for removing the sFLT-1, as well as a carrier for separation having the DNA aptamer immobilized thereto. The present invention provides a DNA aptamer capable of selectively binding to the sFLT-1, including a base sequence containing an artificial base 7-(2-thienyl)-3H-imidazo[4,5-b]-pyridin-3-yl (Ds) at two positions. A carrier for use in an apheresis treatment having the DNA aptamer immobilized thereto is also provided.

## Description

### [Technical Field]

The present invention relates to a DNA aptamer capable of binding to soluble FLT-1 (sFLT-1) and to a use thereof.

### [Background Art]

In recent years, DNA aptamers have attracted attention as a ligand recognition molecule expected to have wide application potential as a nucleic acid pharmaceutical product. In a DNA aptamer, the complementary sequences within a DNA oligonucleotide molecule form a complementary strand, whereby a single-stranded DNA oligonucleotide forms a secondary structure and a tertiary structure. Thus, such a three-dimensional structure allows the DNA aptamer to bind specifically and strongly to a target molecule.

Preeclampsia is a disease that occurs in approximately one out of twenty pregnant women and that causes serious complications in both the mother and the fetus. One of causes of the preeclampsia is an increase in an amount of soluble FLT-1 (soluble fms-like tyrosine kinase-1; hereinafter, referred to as "sFLT-1") in the blood. The sFLT-1 is, as it is also called "sVEGFR1," a soluble form of VEGFR1, and by binding to a VEGF (vascular endothelial growth factor) and a PLGF (placenta growth factor) in the blood, it inhibits the signaling via the VEGFR1, which is their receptor. As a result of such inhibition, it causes inhibition of angiogenesis and vascular endothelial dysfunction, leading to symptoms such as hypertension and proteinuria. Therefore, direct removal of excessive sFLT-1 in the blood is expected to improve the symptoms.

It has been reported, for example, that the sFLT-1 is removed using a dextran sulfate apheresis column that utilizes the properties of basic proteins for the purpose of treating the preeclampsia associated with the sFLT-1 (NPL 1 and PTL 1). As another example of the nonspecific removal of the sFLT-1, it has been reported that the sFLT-1 is removed by simple plasma exchange (NPL 2).

### [Citation List]

### [Patent Literature]

[PTL 1] Japanese Unexamined Patent Application, Publication No. 2016-147065

### [Non Patent Literature]

[NPL 1] J. Am. Soc. Nephrol., 27: 903-913, 2016
[NPL 2] Clinical Nephrology, vol. 96, S101-S106, 2021

### [Summary of Invention]

### [Technical Problem]

In the method using a dextran sulfate apheresis column, since the sFLT-1 and the dextran sulfate bind to each other based on a simple electrostatic interaction, the binding lacks specificity. Therefore, even useful components in the blood may also be erroneously removed by the electrostatic interaction. In addition, components other than the sFLT-1 may adsorb onto the dextran sulfate, saturating the binding sites on the dextran sulfate and reducing the efficiency of the sFLT-1 removal. Thus, the attempt to remove the sFLT-1 using a dextran sulfate apheresis column faces the problem of nonspecific removal of the sFLT-1. Further, the method of removing the sFLT-1 by the simple plasma exchange requires supplementation with a blood preparation accompanying the plasma exchange, and has a potential risk of infection which may be caused by such supplementation.

As a device for specific removal of the sFLT-1, an apheresis column using anti-sFLT-1 antibody conjugated Sepharose (U.S. Patent No. 9,592,331; hereinafter, referred to as "PTL 2") has also been disclosed. However, since this column uses an antibody, there is a problem of heat stability in that autoclave sterilization is not possible, and problems such as a high manufacturing cost due to the method of producing the antibody and variation in the quality between lots may arise.

For these reasons, there is a demand for the development of a substance that can serve as an sFLT-1 adsorbent capable of being immobilized on a column, which is capable of removing the sFLT-1 in the blood more efficiently and specifically, can be manufactured inexpensively, and has uniform quality. From another viewpoint in the treatment of the preeclampsia, anti-VEGF antibody drugs aimed at the inhibition of the VEGFR1 signaling also exist. However, in addition to the aforementioned problems related to the manufacture, antibody drugs present problems such as immunogenicity and placental transfer.

The present invention has been made in view of such circumstances, and an object thereof is to provide a DNA aptamer that can be used as a device for removing the sFLT-1, which is capable of removing the sFLT-1 in the blood more efficiently and specifically, is inexpensive, has uniform quality, and can be used more safely, as well as a carrier having the DNA aptamer immobilized thereto.

### [Solution to Problem]

In order to solve the above problems, a DNA aptamer capable of selectively binding to sFLT-1 and a carrier on which such a DNA aptamer is immobilized according to the present invention employ the following solutions.

A first aspect of the present invention provides a DNA aptamer capable of selectively binding to sFLT-1, including a base sequence containing an artificial base 7-(2-thienyl)-3H-imidazo[4,5-b]-pyridin-3-yl (Ds) at two positions, wherein the base sequence includes 13 to 18 bases between units of Ds at the two positions, and wherein at least one of two units of Ds is located within a sequence forming a single strand upon formation of a tertiary structure.

In the first aspect described above, the DNA aptamer may have a base sequence set forth in any one of SEQ ID NOs: 1 to 9.

In the first aspect described above, the DNA aptamer may have the base sequence set forth in SEQ ID NO: 1, 3, or 4.

In the first aspect described above, (a) the DNA aptamer may have a base sequence set forth in any one of SEQ ID NOs: 2 and 5 to 9; or (b) in the base sequence set forth in any one of SEQ ID NOs: 2 or 5 to 9, a base sequence of three to seven bases, that is linked to a 5' side of a unit of Ds located on the 5' side, among two units of Ds, and a base sequence of three to seven bases from the tenth base counting from a unit of Ds, that is linked to a 3' side of a unit of Ds located on the 3' side, among the two units of Ds, may be arbitrary sequences that form base pairs.

In the first aspect described above, the DNA aptamer may have a base sequence set forth in SEQ ID NO: 7 or 8.

In the first aspect described above, in the DNA aptamer, a base X in the base sequence set forth in SEQ ID NO: 6 or 7 may be an artificially produced base, and the artificially produced base may be modified with a linker, a medium molecular weight compound, a high molecular weight compound, a biological macromolecule, or a polymer having affinity to a biological macromolecule.

A second aspect of the present invention provides a carrier for use in an apheresis treatment, having the DNA aptamer according to the first aspect described above immobilized thereto.

In the second aspect described above, a target of the apheresis treatment using the carrier may be blood positive for sFLT-1.

In the second aspect described above, a disease to which the apheresis treatment using the carrier is applied may be selected from the group consisting of preeclampsia, exudative age-related macular degeneration, central retinal vein occlusion, branch retinal vein occlusion, diabetic macular edema, choroidal neovascularization, malignant tumor, and Duchenne muscular dystrophy.

A third aspect of the present invention provides a material for removing sFLT-1, containing the DNA aptamer according to the first aspect described above.

A fourth aspect of the present invention provides a kit for removing sFLT-1, containing the DNA aptamer according to the first aspect described above.

A fifth aspect of the present invention provides a pharmaceutical composition containing the DNA aptamer according to the first aspect described above.

In the fifth aspect described above, the pharmaceutical composition may be for the treatment and/or the prevention of a disease selected from the group consisting of preeclampsia, exudative age-related macular degeneration, central retinal vein occlusion, branch retinal vein occlusion, diabetic macular edema, choroidal neovascularization, malignant tumor, and Duchenne muscular dystrophy.

### [Advantageous Effects of Invention]

The DNA aptamer capable of selectively binding to sFLT-1 according to the present invention binds specifically and strongly to sFLT-1. Therefore, it enables more efficient and specific removal of the sFLT-1 in the blood.

The DNA aptamer capable of selectively binding to the sFLT-1 according to the present invention can be synthesized by a chemical method. Accordingly, as compared with a synthesis method using a biological technique, it is possible to provide a DNA aptamer with uniform quality at a lower cost.

Furthermore, the DNA aptamer capable of selectively binding to the sFLT-1 according to the present invention makes it possible to provide a carrier for separating the sFLT-1 that is more inexpensive and can be used more safely.

### [Brief Description of Drawings]

[Fig. 1] Fig. 1 is a diagram illustrating results of binding capacity screening by ELOSA for DNAs which are aptamer candidates in one embodiment of the present invention.
[Fig. 2] Fig. 2 is a diagram illustrating results of binding capacity screening by ELOSA for DNA aptamer candidates having modified sequences in one embodiment of the present invention.
[Fig. 3] Fig. 3 is a diagram illustrating results of binding capacity screening by ELOSA for modified DNA sequences in one embodiment of the present invention.
[Fig. 4] Fig. 4 is a diagram illustrating the predicted secondary structure of sequences obtained from the binding capacity screening of the modified DNA sequences.
[Fig. 5] Fig. 5 is a diagram illustrating the secondary structure and variable bases of sequences obtained from a second selection of the modified DNA sequences.
[Fig. 6] Fig. 6 is a diagram illustrating results of binding analysis of the DNA aptamers according to one embodiment of the present invention to sFLT-1 by surface plasmon resonance (SPR), using a C6 linker as a biotin linker.
[Fig. 7] Fig. 7 is a diagram illustrating results of binding analysis of the DNA aptamers according to one embodiment of the present invention to the sFLT-1 by the surface plasmon resonance (SPR), using a TEG linker as the biotin linker.
[Fig. 8] Fig. 8 is a diagram illustrating results of stability analysis of the obtained aptamer candidates in human plasma.
[Fig. 9] Fig. 9 is a diagram illustrating results of experiments for confirming the ability of DNA aptamer-immobilized Sepharose, according to one embodiment of the present invention, to remove recombinant sFLT-1.
[Fig. 10] Fig. 10 is a diagram illustrating results of experiments for confirming the ability of DNA aptamer-immobilized Sepharose, according to one embodiment of the present invention, to remove the sFLT-1 in the serum of patients with preeclampsia.

### [Description of Embodiments]

DNA aptamers have the following advantages: (1) since they are obtained by chemical synthesis, they have a low risk of biological contamination; (2) they are generally low in antigenicity; (3) since they are DNAs, they exhibit sufficient stability at room temperature under conditions nearly neutral and free from nucleic acid degrading enzyme (nuclease); and (4) since they exhibit little inhibitory activity against a drug metabolizing enzyme cytochrome P450, they do not affect concomitant medications. Accordingly, their utility is expected. Furthermore, DNA aptamers do not induce a problem of the production of antibodies against an antibody drug, which is one of possible problems caused when a long-term treatment with the antibody is required, and therefore, aptamers are molecules expected to allow long-term administration.

The present inventors attempted to screen for DNA aptamers as separation materials capable of selectively separating the sFLT-1 from the blood by selectively binding to the sFLT-1. A DNA aptamer is a ligand molecule in which complementary sequences within a DNA oligonucleotide molecule form a complementary strand, causing the single-stranded DNA oligonucleotide to form a secondary structure and a tertiary structure, and the three-dimensional structure thereby binds specifically and strongly to a target molecule. By binding a DNA aptamer having a particular sequence, the activity of the target molecule can be inhibited, suppressed, or enhanced, and the target molecule can be selectively detected or extracted. In recent years, DNA aptamers have attracted attention as such molecules. DNA aptamers have a lower molecular weight than antibodies, being approximately one-tenth or less of the molecular weight of antibodies, yet they have high affinity comparable to antibodies, and exhibit high target selectivity. Accordingly, selective binding agents for the sFLT-1 that exploit the properties of DNA aptamers can minimize the occurrence of side effects due to off-target binding. Furthermore, since the DNA aptamers can be produced by chemical synthesis, they are suitable modalities that can overcome the manufacturing challenges associated with antibodies having a similar functionality such as target specificity.

In this specification, the term "target molecule" refers to a substance to which a DNA aptamer may bind, and in the present invention, the sFLT-1 is the target molecule. The expression "selectively binding to sFLT-1" in this specification means that the DNA aptamer according to the present embodiment binds specifically and strongly to the target substance. In one embodiment of the present invention, one example of an indicator showing that a DNA aptamer "selectively binds to sFLT-1" is a value of the dissociation constant (KD). It is preferable that the KD of a DNA aptamer capable of selectively binding to the sFLT-1 according to the present invention be one-digit nM or less, which is the KD value typical of antibodies. In this specification, "KD" refers to the dissociation constant expressed as k_{off} (dissociation rate) / kₒₙ (association rate). The smaller the KD value is, the stronger the affinity for the target molecule is, and the smaller the k_{off} value is, the less likely the aptamer is to dissociate after binding to the target molecule.

The inventors obtained a plurality of candidate DNA aptamer sequences capable of binding to the sFLT-1 by a SELEX method using a DNA library containing an artificial base 7-(2-thienyl)-3H-imidazo[4,5-b]-pyridin-3-yl (hereinafter, also referred to as "Ds"). From these candidate sequences, selection was performed based on the binding strength to the sFLT-1 in an ELOSA system. The selected sequences were subjected to truncation and addition of mini-hairpin sequences to confirm the binding capacity and the stability in the blood, and anti-sFLT-1 aptamer sequences were identified. Using Sepharose beads having these DNA aptamers immobilized thereon, the ability to remove the sFLT-1 from inactivated FBS spiked with recombinant sFLT-1 at an equivalent amount to that of the sFLT-1 in the blood of preeclampsia patients was examined. The results revealed that the sFLT-1 was removed in an sFLT-1 aptamer-dependent manner, leading to the completion of the present invention. Methods for treating preeclampsia using an sFLT-1 aptamer have not yet been established.

The DNA aptamer according to the present disclosure can provide a device for removing the sFLT-1 for the purpose of treatment of a disease in which the sFLT-1 is involved, such as preeclampsia. Furthermore, when the DNA aptamer according to the present disclosure is administered to the body, it may also be applied as a therapeutic agent for various diseases associated with the signaling of the VEGFR1, which corresponds to the membrane-bound form of the sFLT-1.

A comparison was attempted between the method of removing the sFLT-1 using the DNA aptamer according to the present disclosure and the method (antibody) for specific removal of the sFLT-1 described in the PTL 2. In the Example 1 of the PTL 2, an experiment was performed to remove the sFLT-1 from a 1 mL sample of amniotic fluid derived from a preeclampsia patient containing 40 ng of sFLT-1, using a column containing 0.1 mL of agarose beads with 500 µg of anti-sFLT-1 antibody immobilized. According to the results of the Example 1 in the PTL 2, as a result of confirming the sFLT-1 removal rate after three passes through the column, it is 87% for the most efficient antibody species (antibody 103). In addition, as described in the Example 3 of the PTL 2, in an experiment using an antibody species AG10B, the best result of the removal rate from horse serum spiked with 40 ng of sFLT-1 is shown to be 98%. However, the performances cannot be directly compared with those shown in the removal experiment using sFLT-1 aptamers in the Example 8 of the present embodiment, because the experimental conditions are different. Moreover, in a method using an anti-sFLT-1 antibody, such as the method of the PTL 2, disruption of the sFLT-1/PLGF balance due to excessive removal of the sFLT-1 has been reported to cause adverse effects such as arteriosclerosis. As mentioned above, when comparing the performance of aptamers with that of antibodies, aptamers are more suitable as adsorbents used in apheresis. Furthermore, since the performance of aptamers also include low placental permeability, it is believed that the use of aptamers is preferable in view of administration to pregnant women.

In the present disclosure, the term "naturally occurring base" refers to a nucleic acid base possessed by a nucleotide existing in nature, namely any one of adenine, guanine, cytosine, and thymine.

In the present disclosure, the terms "artificially produced base" or "artificial base" refer to a nucleic acid base that does not exist in nature and is produced by an artificial method. One example of the artificial base is 7-(2-thienyl)-3H-imidazo[4,5-b]-pyridin-3-yl (hereinafter, referred to as "Ds") included in the sequence of the DNA aptamer according to the present embodiment.

In the present disclosure, the term "mini-hairpin sequence" refers to a base sequence consisting of seven to 14 bases, which can increase resistance to degradation by a nucleic acid degrading enzyme, or increase the Tm value of a DNA aptamer having the mini-hairpin sequence attached thereto, thereby enhancing the thermal stability of the DNA aptamer (see, for example, PCT International Publication No. WO 2016/143700).

The DNA aptamer according to the present embodiment is an oligonucleotide having a base sequence containing an artificial base 7-(2-thienyl)-3H-imidazo[4,5-b]-pyridin-3-yl (Ds) at two positions, and is a molecule capable of selectively binding to the sFLT-1. In the present embodiment, a DNA oligonucleotide having the base sequence shown in Table 1 can be used as a DNA aptamer binding to the sFLT-1.

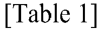

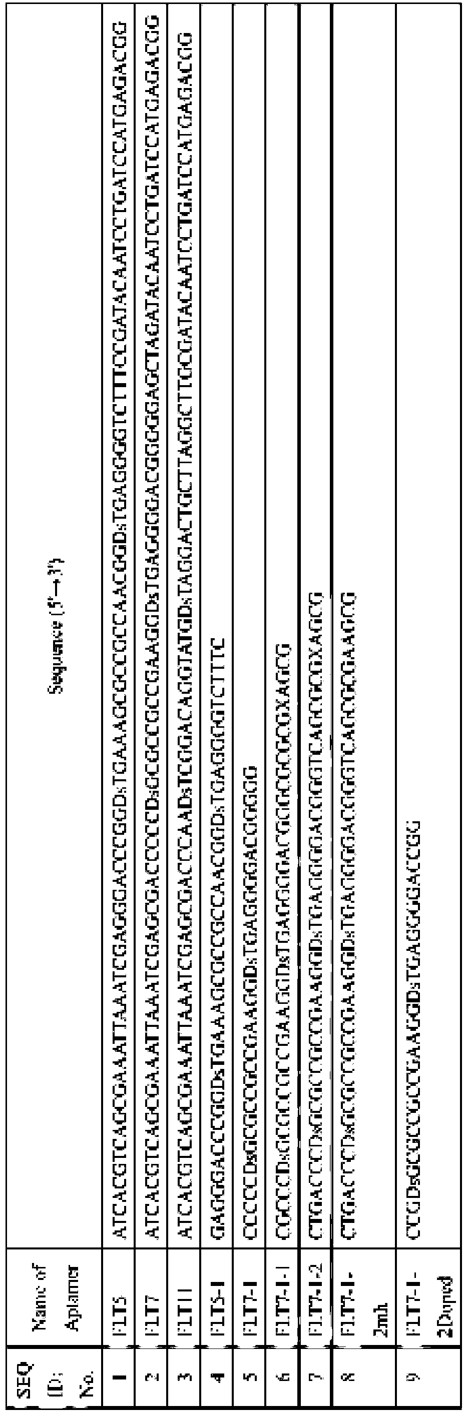

Any one of the base sequences shown in Table 1 include a sequence of 13 to 18 bases between units of Ds at the two positions. The base sequences of SEQ ID NOs: 2 and 5 to 7, which contain "FLT7" in their names of aptamer, include a common sequence of 13 bases between two units of Ds at the two positions. Furthermore, any one of the base sequences of SEQ ID NOs: 2, 5, and 6 include a common sequence of three bases on the 5' side of the above 13 bases, and a common sequence of 12 bases on the 3' side, thereby having a total common sequence of 30 bases. The base sequences of SEQ ID NOs: 1 and 4, which contain "FLT5" in their names of aptamer, include a common sequence of 18 bases between the two units of Ds at the two positions. In the aptamers having the base sequences shown in Table 1, at least one of the two units of Ds is located within the sequence that forms a single strand when tertiary structure is formed.

In the sequences of SEQ ID NOs: 6 and 7, the fifth base from the 3' terminus is substituted with an arbitrary base X. The base X represents either an arbitrary non-naturally occurring base or an artificially produced base, or an artificially produced base conjugated, via a linker, with a low molecular weight compound, a high molecular weight compound (biological macromolecule) used in an organism, such as a peptide, an oligonucleotide, an oligosaccharide, and a protein, or a polymer having biocompatibility (biopolymer). In the present specification, the term "non-naturally occurring base" refers to a nucleic acid base having a structure different from that of naturally occurring bases existing in nature.

The base sequences of SEQ ID NOs: 6 to 8 each include a mini-hairpin sequence consisting of nine bases on the 3'-terminal side. In the base sequences of SEQ ID NOs: 6 and 7, the base X is located within the mini-hairpin sequence. Since the functional molecules described above, such as a low molecular weight compound and a biological macromolecule, are bound via a linker to the base X located within the mini-hairpin, when the DNA aptamer forms the tertiary structure, such functional molecules can be introduced without interfering with a site having the binding activity of the DNA aptamer to the target substance.

The base sequence set forth in any one of SEQ ID NOs: 2 and 5 to 9 may be exactly the sequence as listed in Table 1. Alternatively, in the sequence set forth in any one of SEQ ID NOs: 2 or 5 to 9, a base sequence of three to seven bases, that is linked to a 5' side of a unit of Ds located on the 5' side, among two units of Ds, and a base sequence of three to seven bases from the tenth base counting from a unit of Ds, that is linked to a 3' side of a unit of Ds located on the 3' side, among the two units of Ds, may be arbitrary sequences that form base pairs. For example, in the base sequence of SEQ ID NO: 5, the sequence "CCCCC" that is linked to the 5' side of the unit of Ds located on the 5' side, among the two units of Ds, and the sequence "GGGGG" corresponding to the 10th to 14th bases counting from a unit of Ds, that is linked to the 3' side of a unit of Ds located on the 3' side, among the two units of Ds, form base pairs. In this relationship where the five bases of each sequence form base pairs, that is, the C-G pairs, one or more of the C-G pairs may be substituted with G-C, A-T, or T-A pair(s).

The DNA aptamers having the base sequence listed in Table 1 in this specification may be an oligonucleotide of which the full length corresponds to the base sequence shown in the table. Alternatively, for example, for the purpose of subjecting it to a surface plasmon resonance analysis, a predetermined base sequence may be added to the 5' or 3' termini of the base sequences listed in Table 1.

Regarding a method of producing the DNA aptamer of the present embodiment, it is possible to synthesize the DNA aptamer by a method known in the art. For example, the DNA aptamer according to the present embodiment can be chemically synthesized by a known solid-phase synthesis method. The synthesized DNA aptamer is preferably purified before use by a method known in the art. Examples of a purification method include, but are not limited to, the gel purification method, the affinity column purification method, the HPLC method, and the like.

It is preferable that the base X in the sequence of the DNA oligonucleotide described above be an artificially produced base. Regarding an active functional group to be provided by the introduction of the artificial base X, it is possible to introduce an amino group, a thiol group, a carboxyl group, an azido group, a hydroxyl group, an alkenyl group, a formyl group, a hydrazide group, a cyano group, or the like by selecting an appropriate artificial base. These active functional groups can be used, either as such or after activation, to anchor the DNA aptamer to a carrier usable in an apheresis column.

In the DNA aptamer according to the present embodiment, one end of a hydrocarbon chain linker may be bound to the base X, while the other end may be bound to a carrier, thereby holding the DNA aptamer on the carrier. In the DNA aptamer according to the present embodiment, it is preferable that the base X be a base obtained by modifying T (thymine) or A (adenine).

To the active functional group introduced by the base X, a biocompatible polymer, such as PEG (polyethylene glycol), POX (poly(2-oxazoline)), and PMPC (poly(2-methacryloyloxyethyl phosphorylcholine)), or a medium molecule or a biological macromolecule, such as a peptide, an oligonucleotide, or a polysaccharide, can be conjugated. An aptamer in which its properties have been modified by such conjugation of the biocompatible polymer, the medium molecule, or the biological macromolecule may not only be applied to an apheresis column but also serve as a therapeutic agent for a disease that can be treated by inhibiting the function of the sFLT-1.

Examples of the functional group usable for the conjugation with the medium molecule or the biological macromolecule include an azido group (-N₃), an amino group (-NH₂), a carboxyl group (-COOH) or an active ester thereof, an alkynyl group (-CC) or a cyclic structure containing an alkynyl structure, a formyl group (-CHO), a hydrazide group (-NH-NH₂), a hydroxyl group (-OH), a thiol group (-SH), a cyano group (-CN), a vinyl group (-CHCH₂), and a maleimide group.

Examples of the high molecular weight compound include polyethylene glycol (PEG) having a molecular weight of 20,000 or more and 60,000 or less, or any high molecular weight molecule with biocompatibility having a molecular weight of 20,000 or more. The term "biocompatible polymer" refers to a polymer that is not normally used in an organism but is safe in that it does not cause inflammation or toxic reactions when introduced into an organism. Examples of the medium molecular weight compound include peptides, oligonucleotides, oligosaccharides, proteins, PEG, or any biocompatible polymer, having a molecular weight of more than 1,000 and less than 20,000.

The DNA aptamer according to the present embodiment is immobilized on a carrier and is used for an apheresis treatment. In the present embodiment, the carrier of any material that is stable in an organism and has biocompatibility, such as surface-modified silica gel, surface-modified metals, glass, zeolite, polystyrene, polypropylene, polyethylene, acetylated cellulose, and agarose, may be used for the application of apheresis. Regarding the form of the carrier, beads, fibers, woven fabrics, nonwoven fabrics, porous structures, or the like may be used.

When immobilizing the DNA aptamer according to the present embodiment on a carrier, the immobilization can be carried out at any position on the aptamer, as long as its activity is not impaired, and is not limited to immobilization using the base X that is an artificial base.

As an example of application of the carrier having the DNA aptamer according to the present embodiment immobilized thereto, it is expected that an effect to remove the sFLT-1 from the blood positive for the sFLT-1 is exhibited using an apheresis column obtained by immobilizing the DNA aptamer of the present embodiment to an appropriate carrier with biocompatibility. As a pharmaceutical composition, the DNA aptamer according to the present embodiment may be administered either unmodified or modified by conjugating a macromolecule using the base X in the mini-hairpin structure, by local or systemic administration. Thus, effects to alleviate or suppress a symptom, or therapeutic effects against a disease can be expected. The pharmaceutical composition of the present embodiment can be administered by an appropriate method, as long as the active ingredient is not inactivated. Examples of such a method may include parenteral administration, such as injection, aerosol, topical application, ocular administration, or nasal administration, and oral administration.

A disease to which the apheresis treatment using the carrier according to the present embodiment is applied may be selected from the group consisting of preeclampsia, exudative age-related macular degeneration, central retinal vein occlusion, branch retinal vein occlusion, diabetic macular edema, choroidal neovascularization, malignant tumor, and Duchenne muscular dystrophy.

At present, commercially available dextran sulfate apheresis columns achieve an average removal rate of 18% for the sFLT-1 in the plasma of a patient with preeclampsia, and when such a commercially available product is used, certain effects such as reduction of urinary protein and prolongation of pregnancy period have been observed. However, it has been reported that the plasma level of the sFLT-1 rises again immediately after the treatment with the commercially available product (NPT 1). Therefore, it is believed that frequent treatments are required when such a commercially available product is used.

In contrast, in the apheresis column carrying the DNA aptamer of the present embodiment, it has been confirmed that 50% or more of the sFLT-1 can be removed from the serum of a preeclampsia patient. By using beads having the DNA aptamer according to the present embodiment immobilized thereto, the sFLT-1 can be removed more efficiently in a single treatment, thereby providing a therapy with more sustained and enhanced therapeutic effects. Details will be described in the Examples below.

Since the DNA aptamer according to the present embodiment is capable of selectively binding to the sFLT-1, it can be used as a research reagent for diseases associated with the sFLT-1. For example, for evaluation of the possibility that the sFLT-1 is involved in a physiological phenomenon of interest, tests can be conducted, either in vitro or in vivo, by allowing the DNA aptamer according to the present embodiment to act, thereby enabling consideration of the cause of the physiological phenomenon. Furthermore, the DNA aptamer according to the present embodiment can be used in a wide variety of tests involving a reaction system which inhibits the sFLT-1, by being added as a reagent to a cell culture medium or administered to an animal.

The DNA aptamer capable of selectively binding to the sFLT-1 according to the present embodiment binds specifically and strongly to the sFLT-1. Accordingly, as compared with a conventional nonspecific removal method, it enables more efficient and specific removal of the sFLT-1 in the blood.

Furthermore, the DNA aptamer capable of selectively binding to the sFLT-1 according to the present embodiment can be synthesized by a chemical method. As compared with a synthesis method using a biological method, a chemical synthesis method does not require the use of serum or the like for production. Therefore, the aptamer can be provided at a lower cost and with uniform quality, without causing a problem inherent in the manufacture of an antibody drug, such as a risk of biological contamination or quality variations between lots.

The DNA aptamer capable of selectively binding to the sFLT-1 according to the present embodiment and a carrier having the DNA aptamer immobilized thereto can be stored in a dry state or at room temperature. Therefore, as compared with a carrier having a protein ligand such as an antibody immobilized thereto, the present invention increases the convenience in terms of reduced costs for the transportation and the storage.

### [Examples]

The DNA aptamers used in the present Examples were chemically synthesized according to the methods described in PCT International Publication No. WO 2013/073602 and PCT International Publication No. WO 2016/143700. The DNA aptamers having the base sequence including the artificial base X according to the present Examples were synthesized by introducing, at the positions of X in the sequences of SEQ ID NOs: 6 and 7, dT amidite modified via a linker, using the methods described in PCT International Publication No. WO 2013/073602 and PCT International Publication No. WO 2016/143700. Other X-substituted variants can be synthesized by using commercially available amidites of artificial bases or modified bases.

### <Example 1: Selection of DNA Aptamers Capable of Binding to sFLT-1 Using a Ds-Predetermined DNA Library>

A DNA library containing the artificial base Ds was prepared in accordance with the predetermined method described in PCT International Publication No. WO 2013/073602. This DNA library is composed of DNAs containing sequences having 92 bases in total, consisting of primer sequences arranged at both the 3' and 5' termini, a tag sequence, a fixed sequence, and an intermediate portion with a random sequence. The artificial base Ds is incorporated at two positions within the random sequence in the intermediate portion, and the positions where the units of Ds are incorporated can be identified based on the tag sequence. Sub-libraries having individual tag sequences were chemically synthesized and mixed to prepare the DNA library. Protein-SELEX against the sFLT-1 was performed on a 1 mL scale using 5000 pmol of the above library as a starting material. As the target protein, the sFLT-1 with an Fc tag or the sFLT-1 with a His tag (both available from Sino Biological) was used depending on the round. After completion of seven rounds of selection, the obtained DNAs were subjected to a sequence analysis.

### <Example 2: Sequencing of DNAs Obtained by Protein-SELEX>

The base sequences of the DNAs obtained after seven rounds were identified by Ion PGM sequencing (Thermo Fisher Scientific). From 428,716 sequences, that is the total number of reads, sequences that correctly held the primer sequences at the both termini were extracted, resulting in 134,637 sequences to be analyzed. Based on the extracted sequences, clustering was performed under conditions in which sequences differing by two bases were regarded as derivative sequences, and the degree of enrichment of sequences was examined. As a result, five sequences were identified as candidate binding sequences. Each of these candidate sequences was chemically synthesized and individually subjected to screening for binding capacity.

### <Example 3: Screening for Binding Capacity of Candidate Sequences (ELOSA)>

The five candidate sequences were subjected to screening by a binding capacity analysis using the ELOSA method with the sequences immobilized on plates. In detail, 5 pmol of biotin-labeled sequence was added to a Neutravidin-coated plate (Thermo Fisher Scientific) and immobilized by shaking it at room temperature for 30 minutes. Then, inactivated FBS (fetal bovine serum) was added to the wells containing the immobilized sequences and the plate was shaken at room temperature for 30 minutes to perform blocking. After the blocking, 400 nM of His-tagged sFLT-1 recombinant protein was added, and a binding reaction with the candidate sequence was allowed to proceed at room temperature for 30 minutes. Thereafter, washing was performed three times with 1 × D-PBST (0.05 wt% Tween 20). Detection of the sFLT-1 recombinant protein bound to the sequence was performed with an HRP-labeled anti-His-tag antibody (R&D). In detail, the HRP-labeled anti-His-tag antibody diluted 1000-fold in inactivated FBS was added, shaking was performed at room temperature for one hour under light shielding. Then, washing was performed three times with 1 × D-PBST (0.05 wt% Tween 20). The Substrate Reagent (R&D) was added, and the reaction was allowed to proceed at room temperature for 20 minutes under light shielding. Thereafter, 2N H₂SO₄ was added to quench the reaction, and binding between the candidate sequence and the His-tagged sFLT-1 was detected by measuring the absorbance at 450 nm (OD450).

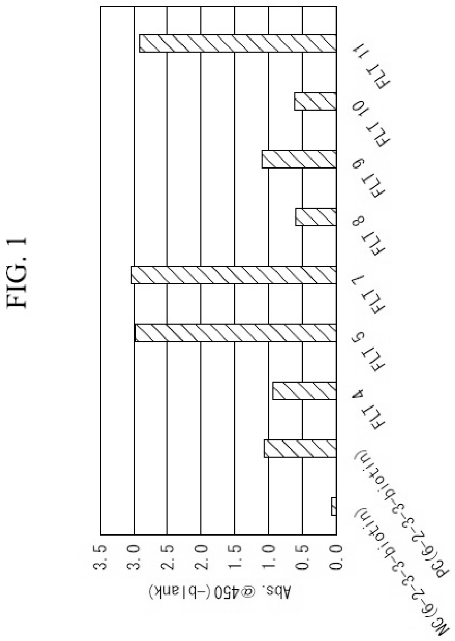

The OD450 measurement values are shown in Fig. 1. In Fig. 1, "NC (6-2-3-3-biotin)" denotes a negative control having the sequence shown below, and "PC (6-2-3-2-biotin)" denotes a positive control having the sequence shown below. As a result of the analysis, it was revealed that the FLT5 (SEQ ID NO: 1), the FLT7 (SEQ ID NO: 2), and the FLT11 (SEQ ID NO: 3) exhibited binding capacities of more than twice as high as that of the positive control sequence.
Name of Aptamer: 6-2-3-3-biotin
   5'-GCACCCAADsTGATTGATCGTGCGGCCTTTAGGDsTTAGAGGCC-3'
Name of Aptamer: 6-2-3-2-biotin
   5'-CACCCAADsTGATTGATCGTGTGTCCTTTAGGDsTTAGAGGGCG-3'

### <Example 4: Modification of Candidate Sequences>

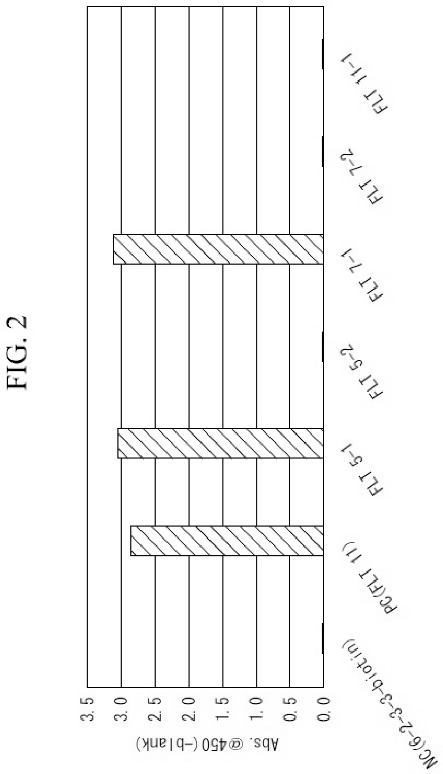

For the candidate sequences FLT5 and FLT7, which exhibited high binding capacities in the Example 3, truncation and modification by addition of a mini-hairpin sequence were carried out. For the candidate sequences that showed high binding capacities to the His-tagged sFLT-1, truncation was carried out, and their binding capacities to the His-tagged sFLT-1 were confirmed similarly to the ELOSA method as described in the Example 3. The results are shown in Fig. 2. As the negative control, the 6-2-3-3-biotin used in the Example 1 was employed, and as the positive control, the FLT11 was employed. As a result of the analysis, it was revealed that the FLT5-1 (SEQ ID NO: 4) and the FLT7-1 (SEQ ID NO: 5) retained binding capacities comparable to that of the FLT5 and the FLT7, respectively, before the modification.

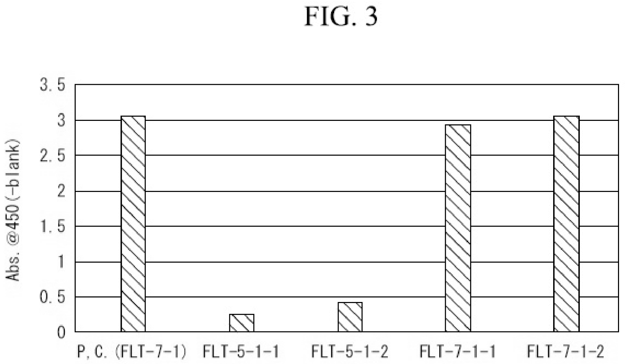

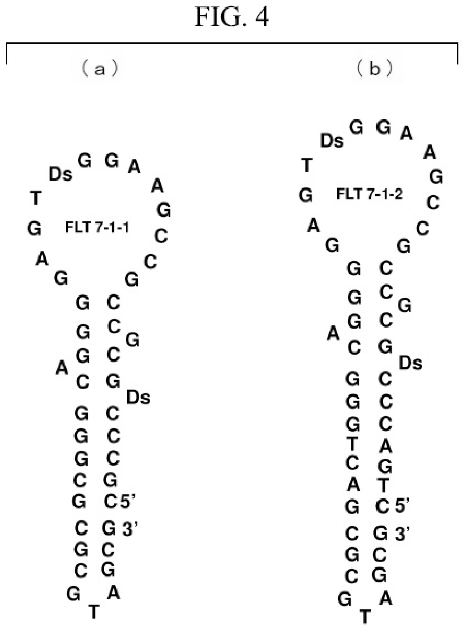

Next, based on the prediction of the secondary structure, further truncation and addition of the mini-hairpin were carried out. For each of the FLT5-1 and the FLT7-1, two types of sequences with different stem lengths were synthesized. The truncated sequences were immobilized on plates at 5 pmol, and were confirmed for the binding capacity to the His-tagged sFLT-1, similarly to the ELOSA method as described in the Example 3. However, the concentration of the recombinant sFLT-1 was changed from 400 nM in the Example 3 to 200 nM for the confirmation of the binding capacity. The results are shown in Fig. 3. The FLT7-1-1 (SEQ ID NO: 6) and the FLT7-1-2 (SEQ ID NO: 7), which are modifications of the FLT7-1, retained binding capacities comparable to that of the unmodified sequence FLT7-1 used as the positive control. The predicted secondary structures of the FLT7-1-1 and the FLT7-1-2 are shown in Fig. 4(a) and Fig. 4(b), respectively.

### <Example 5: Second Selection of DNA Aptamers Obtained by Protein-SELEX>

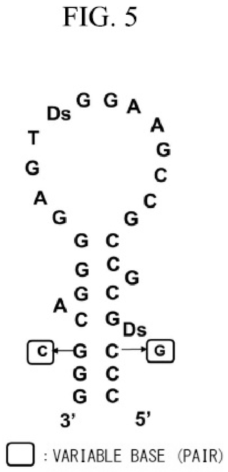

For the DNA aptamer FLT7-1-2 including the sequences obtained in the Example 4, a second selection (Doped selection) was performed in accordance with the method described in the Example 3 of PCT International Publication No. WO 2013/073602, and the secondary structure of the DNA aptamer was estimated. In the second selection, a library prepared at a ratio containing 55% of each base composition of the original sequence obtained in the first selection and 15% of each of the other nucleotides was used to perform selection again. In the selection, only the His-tagged sFLT-1 was used, and four rounds were carried out on a 1 mL scale using 5,000 pmol of the library as the starting material. The predicted secondary structures of the sequences obtained by the Doped selection are shown in Fig. 5. As in the Example 2, an NGS analysis was performed using Ion PGM sequencing (Thermo Fisher Scientific), and the top six sequences with high enrichment rates obtained from the analysis results were individually synthesized and subjected to the binding analysis. As a result, the variable base (pair) was found to be only one position of the base pair, consisting of the third base from the 5' terminus and the third base from the 3' terminus. The sequence of SEQ ID NO: 9 is a sequence in which the base C at the third position from the 5' terminus is substituted with G, and the base G at the third position from the 3' terminus is substituted with C.

### <Example 6: Binding Analysis of DNA Aptamer (FLT7-1-2) to sFLT-1>

The binding capacity of the DNA aptamer FLT7-1-2 obtained in the Example 4 was measured by surface plasmon resonance (SPR) using the Biacore T200 (GE Healthcare). A biotin-labeled dT was introduced at the "X" position in the aptamer sequence. The DNA aptamer was prepared by chemical synthesis. For the synthesis, two types of linkers for coupling with biotin, namely a C6 linker and a TEG (triethylene glycol) linker, were selected, and aptamers containing each were prepared.

The biotin-labeled DNA aptamer was mixed in D-PBS, folded (reconstituted) by heating at 95°C followed by rapid cooling, and prepared. Then, an SA chip (Cytiva) coated with streptavidin was used as the SPR sensor chip, and after irreversibly immobilizing the biotin-labeled DNA aptamer on the chip, binding to the sFLT-1 was analyzed. Note that the SPR measurement was performed under conditions with a running buffer (D-PBS, 0.05% Tween 20) and a set temperature of 25°C.

For immobilization of each DNA aptamer onto the sensor chip, the DNA solution diluted with the D-PBS solution to 6.25 nM was subjected to a folding process (heat denaturation at 95°C for 5 minutes followed by rapid cooling on ice), and then Tween 20 was added to a final concentration of 0.05%. The DNA solution was injected at a flow rate of 5 µL/min for 2.1 µL (corresponding to 25 seconds) to immobilize it on the SA chip. Further, after the immobilization, the DNA aptamer non-specifically adsorbed on the SA chip was washed by injecting 50 mM NaOH solution at a flow rate of 20 µL/min (5 µL, five times). Detection of interaction between the immobilized DNA aptamer (FLT7-1-2) and the sFLT-1 was monitored by injecting the sFLT-1 solutions at 0 nM, 1 nM, 2 nM, 4 nM, 8 nM, 16 nM, 32 nM, 64 nM, and 128 nM (diluted with the running buffer). The measurement conditions were with a flow rate of 100 µL/min, a protein injection time of 150 seconds, and a dissociation time of 900 seconds.

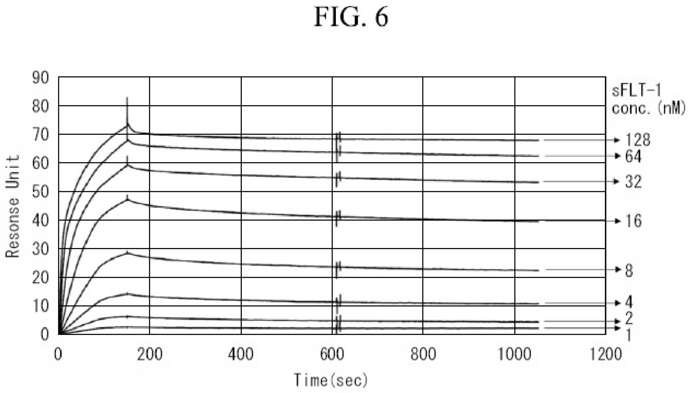

Regeneration of the chip (dissociation of bound protein and refolding of DNA) was performed by injecting 25 µL (corresponding to 15 seconds) of 50 mM NaOH solution, followed by flowing the running buffer for 10 minutes. To subtract response values due to bulk effects on the sensor chip and non-specific adsorption from the sensorgram of each DNA aptamer, a cell without immobilized DNA was used as a reference cell, and its response value was subtracted from the sensorgram of each DNA aptamer. The results obtained using a C6 linker as the biotin linker are shown in Fig. 6. As a result of this measurement, the KD value of the obtained DNA aptamer was 210 ± 0.006 pM.

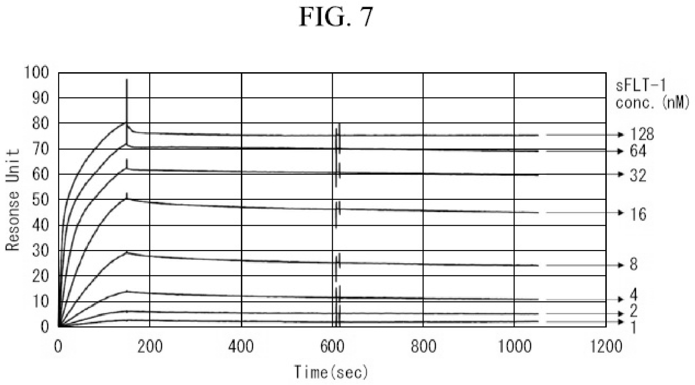

The results obtained using a TEG linker as the biotin linker are shown in Fig. 7. As a result of this measurement, the KD value of the obtained DNA aptamer was 100 ± 0.021 pM.

### <Example 7: Analysis of Stability of DNA Aptamer in Human Plasma>

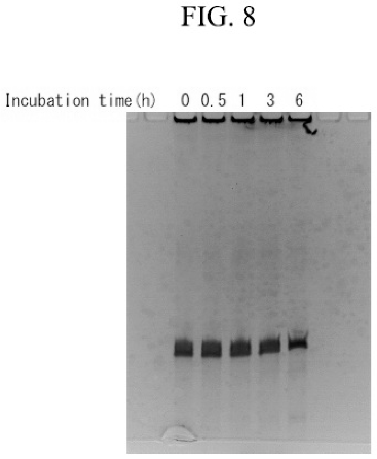

The stability of the aptamer FLT7-1-2mh (SEQ ID NO: 8), in which the fifth base from the 3' terminus of the FLT7-1-2 was substituted with A, against the nucleic acid degrading enzyme contained in human plasma was examined. The DNA aptamer (final concentration: 720 nM) was mixed with human plasma to a final plasma concentration of 96%, and the resulting solution was incubated at 37°C. After 0 hour, 0.5 hour, one hour, three hours, and six hours, 20 µL of the mixed solution was mixed with 110 µL of 1 × TBE and 10 M urea solution to quench the degradation reaction. The samples after the reaction were separated by 7 M urea denaturing 12% polyacrylamide gel electrophoresis, and single-stranded nucleic acids were detected by staining the gel with the SYBR GOLD (Thermo Fisher Scientific). The results of the gel electrophoresis are shown in Fig. 8. From the results of the gel staining, it was revealed that even in the sample after six hours of incubation, almost no band derived from the degradation product was observed and the aptamer remained stable in its full-length form. This suggests that it is also stable and retains its sFLT-1 removal ability during contact with blood samples in apheresis.

### <Example 8: Confirmation of Ability to Remove Recombinant sFLT-1 by Aptamer-immobilized Sepharose>

Beads having an sFLT-1 aptamer immobilized thereto were reacted with inactivated FBS spiked with the recombinant sFLT-1 to confirm the removal rate of the recombinant sFLT-1. In detail, NHS-activated Sepharose beads (Cytiva) were mixed with the sFLT-1 aptamer having an amino linker at the base on the X position of the FLT7-1-2 to prepare the beads having the sFLT-1 aptamer immobilized thereto. The beads having the sFLT-1 aptamer immobilized thereto (100, 200, 300, 400, and 500 µL slurry) were mixed with 1 mL of the inactivated FBS containing the recombinant sFLT-1 (Sino Biological) at an amount equivalent to 8 ng at room temperature for 30 minutes. The amount of the recombinant sFLT-1 was set to be equivalent to the blood concentration thereof in a preeclampsia patient. The amount of the residual sFLT-1 in the supernatant was quantified by the ELISA method.

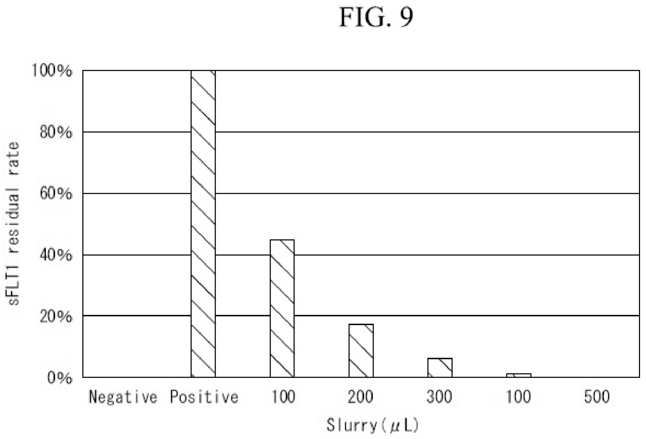

The quantification results are shown in Fig. 9. The amount of the residual sFLT-1 in the supernatant decreased in a bead volume-dependent manner, and it was revealed that, when 500 µL slurry of the beads having the sFLT-1 aptamer immobilized thereto was used, almost the entire amount of the sFLT-1 was removed.

### <Example 9: Confirmation of Ability to Remove sFLT-1 from Preeclampsia Patient Serum by Aptamer-immobilized Sepharose>

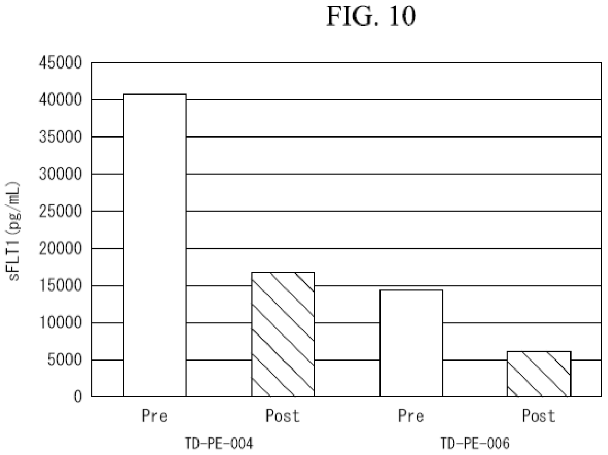

Using the aptamer-immobilized Sepharose described in the Example 8, the removal rate of the sFLT-1 from the preeclampsia patient serum was confirmed. In detail, considering the amount of the sFLT-1 in the patient serum and the possibility that unknown binding inhibitory factors may be present in the serum, the volume of the beads having the sFLT-1 aptamer immobilized thereto was increased to 600 µL slurry, and the beads were mixed with 500 µL of the patient serum at room temperature for 30 minutes. Thereafter, the residual sFLT-1 in the supernatant was quantified in the same manner as described in the Example 8. The results are shown in Fig. 10. It was revealed that approximately 24,000 pg/mL (59%) of the sFLT-1 was removed from the serum sample of Patient 1 (TD-PE-004), and approximately 8,200 pg/mL (57%) of the sFLT-1 was removed from the sample of Patient 2 (TD-PE-006), demonstrating that the aptamer has ability to remove not only the recombinant sFLT-1 but also naturally-occurring sFLT-1.

## Claims

1. A DNA aptamer capable of selectively binding to sFLT-1, comprising a base sequence containing an artificial base 7-(2-thienyl)-3H-imidazo[4,5-b]-pyridin-3-yl (Ds) at two positions, wherein the base sequence includes 13 to 18 bases between units of Ds at the two positions, and wherein at least one of the two units of Ds is located within a sequence forming a single strand upon formation of a tertiary structure.

2. The DNA aptamer according to Claim 1, comprising a base sequence set forth in SEQ ID NO: 1, 3, or 4.

3. The DNA aptamer according to Claim 1, wherein:
(a) the aptamer has a base sequence set forth in any one of SEQ ID NOs: 2 or 5 to 9; or
(b) in the base sequence set forth in any one of SEQ ID NOs: 2 or 5 to 9, a base sequence of three to seven bases, that is linked to a 5' side of a unit of Ds located on the 5' side, among two units of Ds, and a base sequence of three to seven bases from the tenth base counting from a unit of Ds, that is linked to a 3' side of a unit of Ds located on the 3' side, among the two units of Ds, are arbitrary sequences that form base pairs.

4. The DNA aptamer according to Claim 3, comprising a base sequence set forth in SEQ ID NO: 7 or 8.

5. The DNA aptamer according to Claim 3, wherein a base X in a base sequence set forth in SEQ ID NO: 6 or 7 is an artificially produced base, and the artificially produced base is modified with a linker, a medium molecular weight compound, a high molecular weight compound, a biological macromolecule, or a polymer having affinity to the biological macromolecule.

6. A carrier for use in an apheresis treatment, comprising the DNA aptamer according to any one of Claims 1 to 5 immobilized thereto.

7. The carrier according to Claim 6, wherein a target of the apheresis treatment is blood positive for sFLT-1.

8. The carrier according to Claim 6, wherein a disease to which the apheresis treatment is applied is selected from the group consisting of preeclampsia, exudative age-related macular degeneration, central retinal vein occlusion, branch retinal vein occlusion, diabetic macular edema, choroidal neovascularization, malignant tumor, and Duchenne muscular dystrophy.

9. A material for removing sFLT-1, comprising the DNA aptamer according to any one of Claims 1 to 5.

10. A kit for removing sFLT-1, comprising the DNA aptamer according to any one of Claims 1 to 5.

11. A pharmaceutical composition comprising the DNA aptamer according to any one of Claims 1 to 5.

12. The pharmaceutical composition according to Claim 11 for treatment and/or prevention of a disease selected from the group consisting of preeclampsia, exudative age-related macular degeneration, central retinal vein occlusion, branch retinal vein occlusion, diabetic macular edema, choroidal neovascularization, malignant tumor, and Duchenne muscular dystrophy.
